# EUROPEAN PATENT SPECIFICATION

(11) **EP 3 692 169 B1**
(45) Date of publication and mention of the grant of the patent: **16.08.2023**
(21) Application number: 18779703.0
(22) Date of filing: 05.10.2018
(51) Int. Cl.: C12Q 1/6883, G01N 33/68

(54) **BIOMARKERS FOR CHARCOT-MARIE-TOOTH TYPE 2 DISEASE**
BIOMARKER FÜR CHARCOT-MARIE-TOOTH TYP 2 ERKRANKUNG
BIOMARQUEURS POUR LA MALADIE CHARCOT-MARIE-TOOTH TYP 2

(30) Priority: 06.10.2017 EP 17195108
(43) Date of publication of application: 12.08.2020
(73) Proprietor: Universiteit Antwerpen, 2000 Antwerpen (BE); VIB VZW, 9052 Gent (BE)
(72) Inventor: TIMMERMAN, Vincent, 2520 Broechem-Ranst (BE); JUNEJA, Manisha, 2018 Antwerpen (BE)
(74) Representative: Arnold & Siedsma
(86) International application number: PCT/EP2018/077133
(87) International publication number: WO 2019/068865

(56) References cited:
- US-A1- 2007 099 203
- US-B1- 8 753 818
- DESROCHES CARO-LYNE ET AL: "Carrier frequency of guanidinoacetate methyltransferase deficiency in the general population by functional characterization of missense variants in theGAMTgene", MGG - MOLECULAR GENETICS AND GENOMICS, SPRINGER, BERLIN, DE, vol. 290, no. 6, 24 May 2015 (2015-05-24), pages 2163-2171, XP035882002, ISSN: 1617-4615, DOI: 10.1007/S00438-015-1067-X [retrieved on 2015-05-24]
- JUNEJA MANISHA ET AL: "PFN2 and GAMT as common molecular determinants of axonal Charcot-Marie-Tooth disease.", August 2018 (2018-08), JOURNAL OF NEUROLOGY, NEUROSURGERY, AND PSYCHIATRY AUG 2018, VOL. 89, NR. 8, PAGE(S) 870 - 878, XP9509635, ISSN: 1468-330X abstract
- MANISHA JUNEJA ET AL: "Challenges in modelling the Charcot-Marie-Tooth neuropathies for therapy development", JOURNAL OF NEUROLOGY NEUROSURGERY & PSYCHIATRY., 17 July 2018 (2018-07-17), XP055527496, GB ISSN: 0022-3050, DOI: 10.1136/jnnp-2018-318834
- J. M. ANTONY ET AL: "The Human Endogenous Retrovirus Envelope Glycoprotein, Syncytin-1, Regulates Neuroinflammation and Its Receptor Expression in Multiple Sclerosis: A Role for Endoplasmic Reticulum Chaperones in Astrocytes", THE JOURNAL OF IMMUNOLOGY, vol. 179, no. 2, 15 July 2007 (2007-07-15) , pages 1210-1224, XP055283181, US ISSN: 0022-1767, DOI: 10.4049/jimmunol.179.2.1210
- LEE G ET AL: "Differential gene expression in chronic inflammatory demyelinating polyneuropathy (CIDP) skin biopsies", JOURNAL OF NEUROLOGICAL SCIENCES, ELSEVIER SCIENTIFIC PUBLISHING CO, AMSTERDAM, NL, vol. 290, no. 1-2, 15 March 2010 (2010-03-15), pages 115-122, XP026877781, ISSN: 0022-510X [retrieved on 2009-11-17]
- ANDRES R H ET AL: "Functions and effects of creatine in the central nervous system", BRAIN RESEARCH BULLETIN, ELSEVIER SCIENCE LTD, OXFORD, GB, vol. 76, no. 4, 1 July 2008 (2008-07-01), pages 329-343, XP022681005, ISSN: 0361-9230, DOI: 10.1016/J.BRAINRESBULL.2008.02.035 [retrieved on 2008-03-24]

## Description

### FIELD OF THE INVENTION

The present invention in general relates to the diagnosis of neurodegenerative and/or neuromuscular diseases such as Charcot-Marie-Tooth Disease, and is in particular based on the finding of 2 novel biomarkers (PFN2 and GAMT), shown to be downregulated in patients.

### BACKGROUND TO THE INVENTION

Charcot-Marie-Tooth disease (CMT) or hereditary motor and sensory neuropathy (HMSN) is one of the most frequent inherited peripheral neuropathies which is classified into a demyelinating (CMT1) and an axonal form (CMT2) based on where the primary deficit occurs ¹.

Molecular genetic studies have revealed that for the axonal CMT alone, already more than 140 mutations in 26 genes have been identified and the list keeps growing every year ². The diagnosis and prognosis for a given individual involves careful assessment of medical history, physical examination, neurological examination and nerve conduction testing as well as a detailed family history and the use of genetic testing ³. However, the presence of tremendous genetic diversity renders its diagnosis and prognosis rather challenging. Very recently, potential biomarkers for CMT1 have been reported from plasma and skin biopsies derived from CMT1 patients ^{4, 5}. However, no studies have been reported to date describing biomarkers for CMT2. Thus, it remains essential to identify hallmarks of axonal degeneration that are specific for CMT2. Moreover, experimental studies have so far focused on the clinical aspects and/or pathomechanisms for single genes ⁶. Given the genetic heterogeneity of the disease and the fact that it is a rather rare disorder of the peripheral nervous system, this 'single gene' approach may prove impractical clinically. Herein, we intend to identify commonalities among different axonal CMT subtypes, which may reveal novel common biomarkers, and therapeutic targets that will be of benefit for a larger group of CMT patients.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

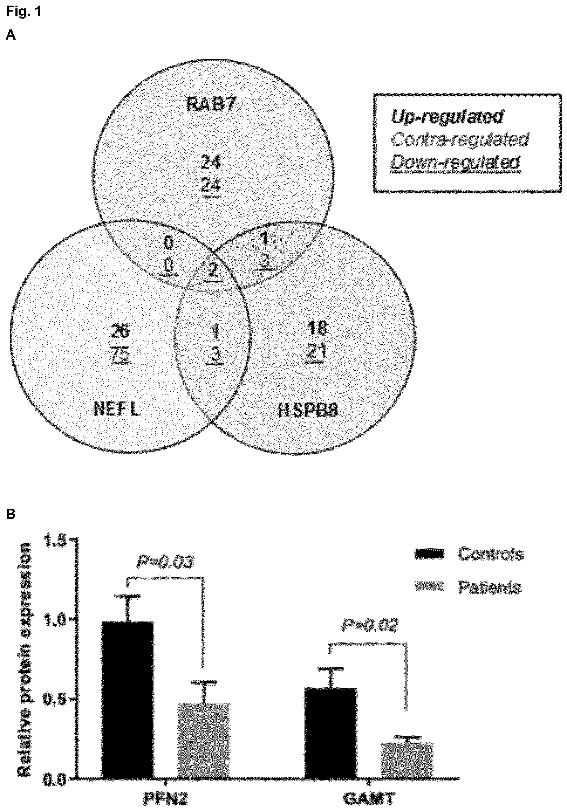

**Figure 1****. Proteomic analysis of lymphoblasts derived from CMT2 patients.**
   (A) Vennplex analysis of significantly differentially regulated proteins (Mean±2SD, Cl:95%) among patients with HSPB8, NEFL and RAB7 mutations as compared to unaffected control individuals. Pooled samples from age and gender matched individuals (3 men and 3 women) were used as controls. (B) Quantification of western blot normalized to β-actin using Image J software.

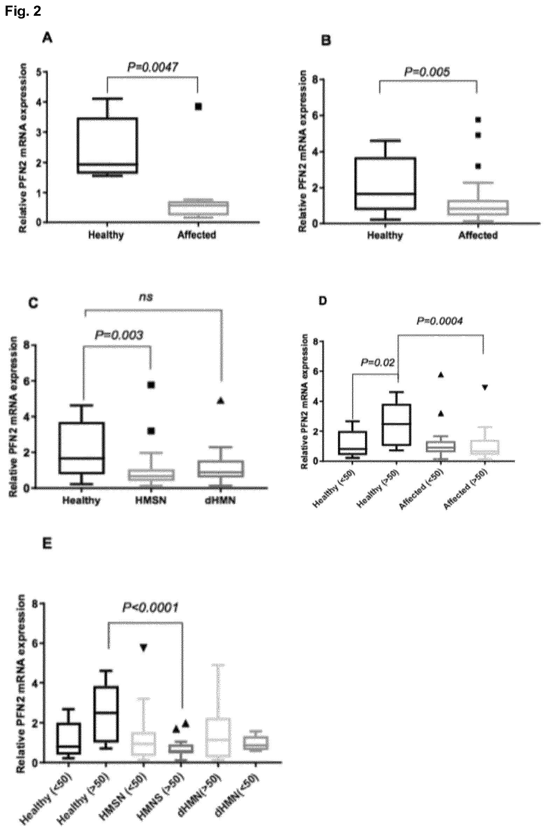

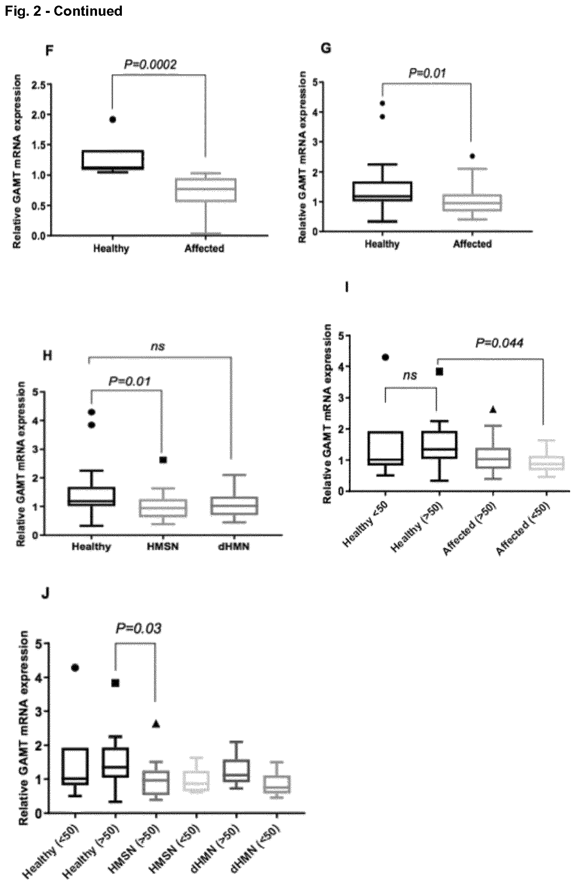

**Figure 2****: mRNA expression levels of PFN2 and GAMT in the CMT2 patient cohort.** (A, F) Relative quantities (ΔΔC_{T}) of PFN2 and GAMT mRNA levels in lymphoblasts that were already available in-house (Belgium cohort). This included 10 CMT2 patient and 6 control samples. (B, G) Relative quantities (ΔΔC_{T}) of PFN2 and GAMT mRNA levels in the entire cohort including 43 CMT2 patient and 22 control samples. (C, H) PFN2 and GAMT mRNA levels in 27 patients with both motor and sensory symptoms (HMSN) and 16 patients with predominantly motor symptoms (dHMN) at the time of diagnosis. (D, I) mRNA expression levels of PFN2 and GAMT in patients classified based on age (cut off = 50 years; 24 patients belong to ≥ 50 years age-group and 19 patients belong to ≤ 50 years age-group whereas 14 controls belong to ≥ 50 age group and 8 controls belong to ≤ 50 years age-group). (E, J) Finally, expression levels of PFN2 and GAMT in patients classified based upon both motor and sensory symptoms as well as age i.e. 15 patients with age ≥ 50 and with both motor and sensory symptoms, compared to data from 14 controls with age ≥ 50. All samples were run in duplicates.

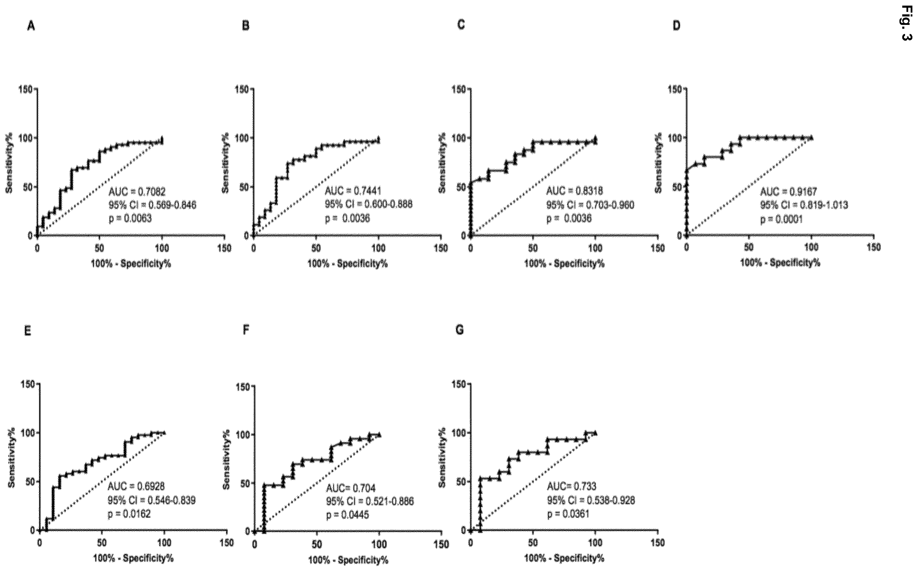

**Figure 3****: Receiver operating characteristic (ROC) curves for the diagnosis of axonal CMT2.** (A) ROC curve for CMT diagnosis using PFN2 expression in the overall patient versus healthy control group. (B) Patients with both motor and sensory deficits (HMSN) versus healthy control group. (C) Patients ≥ 50 years of age versus healthy controls ≥ 50 years of age. (D) HMSN patients ≥ 50 years of age versus healthy controls ≥ 50 years of age. (E) ROC curve for CMT diagnosis using GAMT expression in the overall patient versus healthy control group. (F) Patients ≥ 50 years age versus healthy controls ≥ 50 years age. (G) HMSN patients ≥ 50 years age versus healthy controls ≥ 50 years age. Dotted lines indicate identity (%) and straight lines indicate sensitivity (%), AUC = Area under the curve and Cl = Confidence interval.

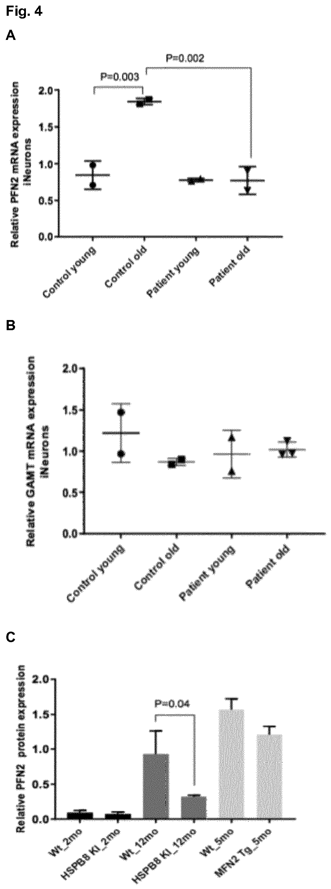

**Figure 4****: PFN2 and GAMT expression in motor neurons**
   (A) Relative quantities (ΔΔC_{T}) of PFN2 and (B) GAMT transcript expression in the motor neurons differentiated from control and patient derived fibroblasts. "Control young" indicates motor neurons derived from healthy individuals (24 years old) whereas "control old" indicates neurons from healthy individuals which are above 50 years old. "Patient young" and "patient old" represent patients either younger than or older than 50 years, respectively. (C) PFN2 expression in sciatic nerve lysates of 2- and 12- month-old HSPB8 KI mice and 5-month-old MFN2-R94Q transgenic (Tg) mice along with their respective wild-type (WT) littermates normalized to α-tubulin using Image J software. Values are expressed as mean ± SEM, n=3.
**Figure 5****: Gender dependent correlation of PFN2 and GAMT**
   Relative quantities (ΔΔC_{T}) of PFN2 (A) and GAMT (B) mRNA levels based on gender in the entire cohort including 43 CMT2 patients (25 females, 18 males) and 22 controls (12 females, 10 males).
**Figure 6****: Age dependent correlation of PFN2 expression**
   A) Correlation of PFN2 transcripts levels with age. Spearman correlation coefficient analysis for PFN2 expression and age of the individual was done for non-parametric data obtained from qRT-PCR analyses of 22 unaffected controls and the correlation coefficient 'r' was calculated. B) Age dependent progression of PFN2 transcripts in patients (n=43) and controls (n=22) reveals a threshold at a mean of 50 years of age. This threshold was used to stratify patients below and above 50 years. Data represent mean±SEM.

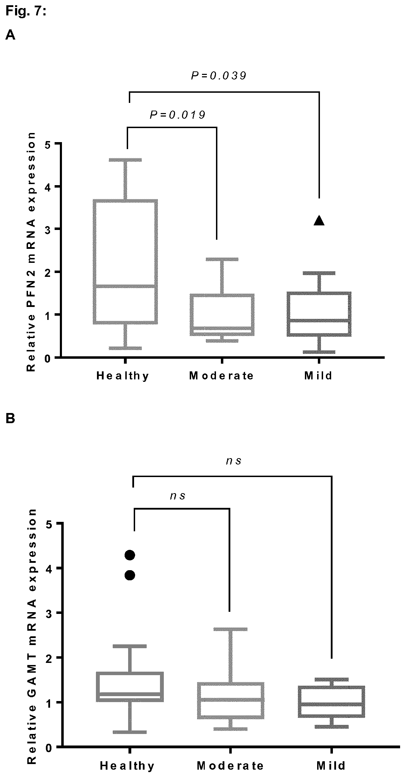

**Figure 7****: PFN2 and GAMT correlation with CMTES scores**
   Relative quantities (ΔΔC_{T}) of PFN2 (A) and GAMT (B) mRNA levels based on CMTES scores in the UK and Italian cohort including 25 CMT2 patients (12 individuals showing moderate symptoms with CMTES ≥ 9 and 13 showing mild symptoms with CMTES ≤ 8) and 22 Belgian controls.

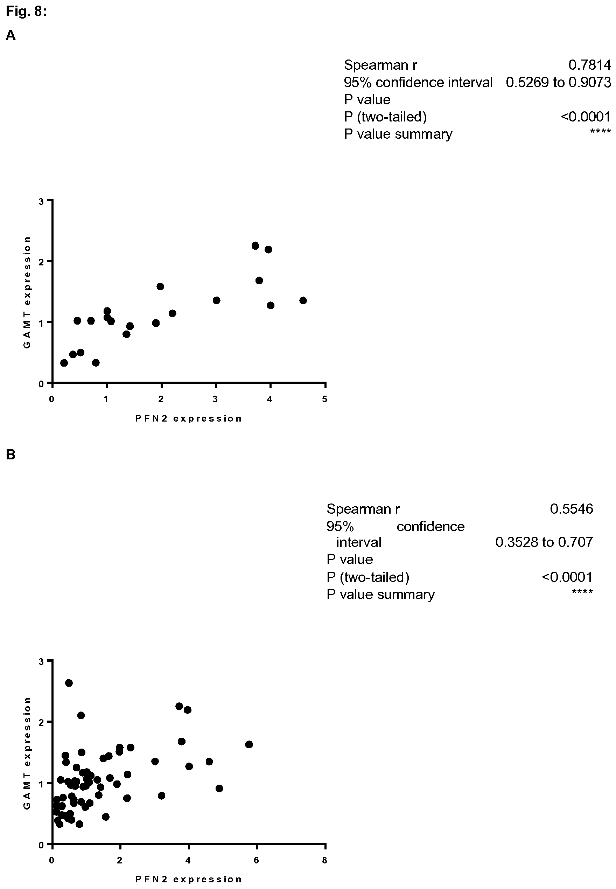

**Figure 8****: PFN2 and GAMT correlation**
   A) The Spearman correlation coefficient 'r' for PFN2 and GAMT expression was calculated for non-parametric data obtained from qRT-PCR analyses of 22 unaffected controls. B) The correlation coefficient 'r' was calculated for both CMT2 patients and controls (n=65).

### SUMMARY OF THE INVENTION

In a specific embodiment, the present invention provides a method for the diagnosis of Charcot-Marie-Tooth disease (CMT) in a subject; said method comprising the steps of:
2) determining the expression level of one or more biomarkers selected from the list comprising: Profilin 2 (PFN2) and guanidinoacetate methyltransferase (GAMT), in a test sample;
3) comparing said expression level of said one or more biomarkers as determined in step 2) with the expression level of said one or more biomarkers in a reference sample;
4) wherein a reduced expression level of said one or more biomarkers in said test sample compared to said reference sample is indicative of said subject suffering from Charcot-Marie-Tooth disease (CMT).

In a particular embodiment of the present invention, said samples are selected from the list comprising a lymphoblast sample or a motor neuron sample obtained from subject-derived iPSC lines; more in particular, said samples are obtained from an individual having an age of at least 50 years.

In another particular embodiment, said Charcot-Marie-Tooth (CMT) disease is selected from the following subtypes: patients diagnosed with hereditary motor and sensory neuropathy (HMSN); or patients with predominantly motor involvement, known as distal hereditary motor neuropathy (dHMN).

In a further embodiment, said test sample is a motor neuron sample obtained from a subject-derived iPSC line from an individual, and said biomarker is selected to be PFN2; alternatively said test sample may also be a lymphoblast sample obtained from an individual, and said biomarker may be selected to be PFN2; alternatively said test sample may be a lymphoblast sample obtained from an individual, and said biomarker may be selected to be GAMT; alternatively said test sample may be a serum sample obtained from an individual, and said biomarker may be selected from PFN2 and GAMT.

More in particular, said test and/or control samples used in the present invention are obtained from an individual having an age of at least 50 years.

In a specific embodiment, the present invention provides an in-vitro use in the claimed method kit for diagnosing Charcot-Marie-Tooth type 2 disease (CMT) in a subject comprising means for determining the expression level of one or more biomarkers selected from the list comprising: Profilin 2 (PFN2) and guanidinoacetate methyltransferase (GAMT).

In a particular embodiment of the methods and kits of the present invention, said expression level is determined from a proteins or mRNA sample.

### DETAILED DESCRIPTION OF THE INVENTION

In the context of the present invention, the phrase 'neurodegenerative and/or neuromuscular disorder' is meant to be a condition which primarily affects the neurons in the peripheral or central nervous system. These disorders are incurable and eventually result in progressive degeneration and/or death of nerve cells, thereby typically causing problems with movement and/or mental functioning. For example, neuromuscular disorders affect the nerves that control the voluntary muscles, such as those in arms and legs, as a result the muscles weaken thereby typically leading to twitching, cramps, pains and joint and movement problems.

In the context of the present invention, said neurodegenerative and/or neuromuscular diseases is Charcot-Marie-Tooth disease (CMT) type 2

Outside the scope of the invention, the HMSN contain next to CMT neuropathies also additional and more complex disease entities such as CMT+ nephropathy, CMT+ optic atrophy, CMT+ skin disease, CMT+ deafness, Giant Axonal Neuropathy (GAN), Silver Syndrome, etc. In respect of amyotrophic lateral sclerosis (ALS), 'juvenile' ALS can sometimes be confounded with distal Hereditary Motor Neuropathy (HMN), also known as 'proximal SMA' or 'spinal form of CMT'. For example, patients with SETX and FIG4 mutations can be diagnosed as severe CMT neuropathy but also as a type of ALS (e.g. ALS4 or juvenile ALS). Moreover, specific frameshift mutations in HSPB1 can cause ALS. Both ALS and distal HMN are axonal neuropathies affecting mainly the motor neurons. Hereditary sensory and autonomic neuropathies (HSAN) often overlap with axonal CMT. Again these peripheral neuropathies affect the axons of sensory neurons, and are associated with loss of pain perception, ulcero-mutilations, congenital insensitivity to pain and anhidrosis (CIPA), etc. For example, RAB7 mutations may be found in CMT2B patients that resemble the HSAN type I phenotype. There are also phenotypic and genetic overlaps between HMSN (including CMT), HMN and HSN neuropathies and hereditary spastic paraplegias (HSP), for example by means of BSCL2, ATL1 and/or ATL3 mutations. Neuromuscular diseases (NMD) (such as Becker or Duchenne muscular dystrophy) is a very heterogeneous group of muscle disorders. Many patients with NMD are also diagnosed as CMT subtypes. For example, BAG3 and HSPB8 mutations can be associated with myofibrillar myopathies, but also distal HMN and axonal CMT neuropathies.

In a particular embodiment, said Charcot-Marie-Tooth type 2 disease is selected from the following subtypes: patients diagnosed with hereditary motor and sensory neuropathy (HMSN); or patients with predominantly motor involvement, known as distal hereditary motor neuropathy (dHMN). ,

In the context of the present invention, the term 'test sample" is meant to be a sample obtained from a subject suspected of having a neurodegenerative and/or neuromuscular diseases, such as Charcot-Marie-Tooth disease (CMT). A 'reference sample' as used in the present invention, it meant to be a sample obtained from a healthy individual, in particular, an individual who is not suffering from a neurodegenerative and/or neuromuscular diseases. The reference sample may also be a pool of samples obtained from healthy individuals, such as for preparing an established standard. Any test or assay conducted according to the invention may thus be compared with an established standard, and it is not necessary to obtain a separate control sample each time for comparison.

In the context of the present invention, typically differential gene expression is identified between the test sample and the reference sample. The terms "differentially expressed gene", "differential gene expression" and their synonyms, which are used interchangeably, refer to a gene whose expression is activated to a higher or lower level in a subject, relative to its expression in a normal or control subject. A differentially expressed gene may be either activated or inhibited at the nucleic acid level or protein level, or may be subject to alternative splicing to result in a different polypeptide product. Differential gene expression may include a comparison of expression between two or more genes, or a comparison of the ratios of the expression between two or more genes, or even a comparison of two differently processed products of the same gene, which differ between normal subjects and subjects suffering from a disease, or between various stages of the same disease. Differential expression includes both quantitative, as well as qualitative, differences in the temporal or cellular expression pattern in a gene or its expression products. As used herein, "differential gene expression" can be present when there is, for example, at least an about a one to about two-fold, or about two to about four-fold, or about four to about six-fold, or about six to about eight-fold, or about eight to about ten-fold, or greater than about 11-fold difference between the expression of a given gene in a patient of interest compared to a suitable control. However, folds change less than one is not intended to be excluded and to the extent such change can be accurately measured, a fold change less than one may be reasonably relied upon in carrying out the methods disclosed herein. In some embodiments, the fold change may be greater than about five or about 10 or about 20 or about 30 or about 40. The term expression level as used in the context of the present invention may include the detection of protein levels, however, alternatively, it may also include determining mRNA expression levels. Expression levels of proteins or mRNA fractions may be determined using any suitable means such as including but not limited to: RT-PCR, western blotting, ELISA, LC-MS/MS,...

In a particular embodiment of the present invention, the test and reference samples are selected from the list comprising a lymphoblast sample or a motor neuron sample obtained from subject-derived iPSC lines; more in particular, said samples are obtained from an individual. Said lymphoblast sample may be obtained by collecting a blood sample from a patient, and subsequently collecting lymphocytes from said sample by any suitable means. A motor neuron sample may be obtained using a method including the steps of isolating primary human fibroblast samples from a skin biopsy from a subject, followed by generating iPSC cell lines therefrom, and differentiating those into spinal motor neurons.

It was the finding of the present invention, that particular genes are suitable for diagnosing the claimed disorders. These genes are Profilin 2 (PFN2) and guanidinoacetate methyltransferase (GAMT). Hence, in a specific embodiment, said test sample is a motor neuron sample obtained from a subject-derived iPSC line from an individual, and said biomarker is selected to be PFN2; alternatively said test sample may also be a lymphoblast sample obtained from an individual, and said biomarker may be selected to be PFN2; alternatively said test sample may be a lymphoblast sample obtained from an individual, and said biomarker may be selected to be GAMT; alternatively said test sample may be a serum sample obtained from an individual, and said biomarker may be selected from PFN2 and GAMT.

In a specific embodiment of the present invention, the test and/or control sample is obtained from an individual having an age of at least 50 years.

In the context of the present invention, the gene PFN2 (or Profilin 2), encodes a protein which is a ubiquitous actin monomer-binding protein belonging to the profiling family. It is thought to regulate actin polymerization in response to extracellular signals. There are 2 alternatively spliced transcript variants encoding different isoforms described for this gene. The gene GAMT (or guanidinoacetate methyltransferase) as used in the context of the invention, encodes a methyltransferase that converts guanidoacetate to creatine, using S-adenosylmethionine as the methyl donor. Defects in this gene have been implicated in neurologic syndromes and muscular hypotonia, probably due to creatine deficiency and accumulation of guanidinoacetate in the brain of affected individuals. Two transcript variants encoding different isoforms have been described for this gene.

In a further aspect, the present invention provides the in-vitro use in the claimed method of a kit for diagnosing Z CMT2 in a subject comprising means for determining the expression level of one or more biomarkers selected from the list comprising: Profilin 2 (PFN2) and guanidinoacetate methyltransferase (GAMT).

In a specific embodiment, the present invention provides a kit for diagnosing Charcot-Marie-Tooth disease type 2 in a subject comprising primers for determining the expression level of both Profilin 2 (PFN2) and guanidinoacetate methyltransferase (GAMT). by RT-qPCR.

In a particular embodiment of the methods and uses of the present invention, said expression level is determined from a protein or mRNA sample.

### EXAMPLES

### Materials and methods

### Participants and clinical characterization

This was a multi-center study involving axonal CMT patients obtained through neuromuscular centers in Belgium (18 patients), Italy (17 patients), and the United Kingdom (8 patients). The study included overall 43 CMT2 patients (19 males and 24 females; median age: 54 years; range: 17-78 years) and 22 healthy controls (18 from Antwerp and 4 from Italy, 10 males and 12 females; median age: 57.5 years; range: 31-71 years). CMT2 being a rare inherited neuropathy, our cohort is to the best of our knowledge, the largest cohort that includes 39 axonal CMT patients harbouring different CMT causal mutations along with different gender and ethnicity. These patients can be further divided into clinically defined subtypes; patients diagnosed with hereditary motor and sensory neuropathy (HMSN) or with predominantly motor involvement, known as distal hereditary motor neuropathy (dHMN) at the time of sample collection. The patient phenotype was defined based on the following clinical and electrodiagnostic inclusion criteria: (1) slowly progressive distal wasting and weakness with reduced or absent reflexes in both upper and lower limb; and (2) predominant motor axonal neuropathy on nerve conduction studies (NCS). As there is a marked phenotypic overlap between dHMN and CMT2, we also included patients who presented with additional clinical features such as lower limb sensory abnormalities (i.e., feet paraesthesia) and sensory abnormalities upon NCS (i.e., decreased lower limbs sensory nerve action potentials [SNAPs] amplitudes). This study was performed according to ethical guidelines and diagnostic procedures issued by the local research ethics committees. Informed written consent was obtained from all patients involved in this study.

### Sample collection, cell culturing and maintenance

Blood samples were collected at the respective hospitals and shipped to us in vacutainers coated with lithium heparin. From these samples, lymphocytes were collected and EBV-transformed to obtain immortalised lymphoblast cell lines. These lines were cultivated at 37°C and 6% CO₂ in Gibco^{®} RPMI 1640 medium supplemented with 15% foetal calf serum. All cell culture media and supplements were purchased from Life Technologies and checked for mycoplasma infection by our cell core facility.

### Human iPSC generation and differentiation into motor neurons

Primary human fibroblast cultures were obtained from skin biopsies of five CMT2 patients after informed consent. Human iPSC lines were generated and characterized by the Stem Cell Institute, KU Leuven, Belgium as previously described (Takahashi *et al.,* 2007). The different iPSC clones were validated for their pluripotency and the presence of the disease associated CMT2 mutation. iPSC lines were then differentiated into spinal motor neurons (iNeurons) as previously described (Maury *et al.,* 2015). The iNeuron cultures were validated by confirming the expression of Choline Acetyltransferase (ChAT) and Islet (Isl1) by immunostaining, and Oligodendrocyte transcription factor (Olig2), Homeobox protein (HB9), Isl1, ChAT, and paired box protein (PAX6) by RT-qPCR. Additionally, we differentiated 4 healthy control iPSC lines (age-matched) for the comparative studies.

### Mass Spectrometry

### Sample preparation and iTRAQ labelling

Total proteins were extracted from lymphoblast cell lines obtained from patients and healthy controls using RIPA lysis buffer (50 mM Tris.HCl; pH 7.5, 150 mM NaCl, 1% Nonidet P-40, 0.1% SDS (sodium dodecyl sulphate) and 0.5% DOC (deoxycholic acid, sodium salt) supplemented with complete protease inhibitor tablets (Roche Diagnostics) for 30 min on ice. Total proteins were precipitated with TCA (tri-chloroacetic acid) followed by a subsequent washing step with acetone. Proteins were resolubilised in buffer (6 M urea, 2 M thiourea, 0.1% SDS, 50 mM TEAB (Triethylammonium bicarbonate); Sigma-Aldrich) and their concentrations were measured. Equal amounts of proteins from each sample were reduced by TCEP [Tris-(2-Carboxyethyl) phosphine, hydrochloride; Sigma-Aldrich) and alkalised by MMTS (5-methylmethanoethiosulphate; Sigma-Aldrich and samples were digested with trypsin at 37°C overnight. Each peptide sample was labelled using iTRAQ reagents (Sciex) as per manufacturer's instruction. The labelled peptides were pooled and dried by SpeedVac (ThermoScientific).

### LC-MS/MS

The mixed peptides were fractionated first on strong cationic exchange chromatography column (1 mm × 150 mm polysulfoethyl aspartamide column (Dionex) and secondly separated on a nano-LC C18 column (200 Å, 2 µm, 75 µm × 25 cm). The nano-LC system (Dionex ULTIMATE 3000) is coupled online to a Q Exactive^{™}-Plus Orbitrap (ThermoScientific) mass spectrometer (MS). The nano-LC eluent was directed with 300 nl/min to a nano-ESI source (with capillary voltage of 1.8 KV) for Orbitrap analysis. The mass spectrometer was set to perform data dependent acquisition (DDA) in the positive ion mode for a selected mass range of 350-1800 m/z at the MS1 level at 140,000 resolution and the MS2 level at 17,500 resolution.

### Data analysis

Proteome Discoverer 2.0 (Thermo Scientific) was used to analyze the raw data using Sequest HT as search engine against the human UniProt/SwissProt database. A threshold of confidence above 99% and a local false discovery rate of less than 1% were used for protein identification. More than one unique peptide was required for protein identification. The identified proteins were delivered in a list containing iTRAQ ratios of expression levels over control sample. These ratios were converted into log scale. To identify differentially regulated proteins, the mean and the 95% confidence limits (2 standard deviations; SD) was calculated. Proteins outside 2 SD's were considered to be significantly altered. The significantly altered proteins from each patient was analysed and compared to other patients using Vennplex program (Cai et al, 2013).

### RNA extraction and real-time quantitative RT-PCR (qRT-PCR)

Total RNA was isolated from patient and control lymphoblasts using the Roboklon kit (Genematrix) according to manufacturer's instructions. RNA was quantified (Nanodrop, ThermoScientific) and 1 µg of RNA was reverse transcribed by using M-MLV Reverse Transcriptase system (Life Technologies) according to manufacturer's protocol. The cDNA was amplified by qPCR using SYBR Green dye chemistry using the ViiA 7 Real-Time PCR system (Applied Biosystems) using primers for profilin2 (NM_053024.3; Fwd: 5'-CGGCAGAGCTGGTAGAGTCTT-3' - SEQ ID N° 1; Rev: 5'- TAGCAGCTAGAACCCAGAGTC-3' - SEQ ID N° 2) and GAMT (NM_000156.5; Fwd: 5'-TGGCACACACACCAGTTCA-3' - SEQ ID N° 3; Rev:5'- AAGGCGTAGTAGCGGCAGTC-3' - SEQ ID N° 4). Data analysis was performed with the qbase+ software (Biogazelle). Mean values were calculated from duplicate RT-qPCR reactions. The RT-qPCR data was normalized according to the method described by Vandesompele et al. (Vandesompele *et al.,* 2002), by geometric averaging of multiple internal control genes.

### Protein extraction and western blotting from human and mouse samples

Hspb8K141N/K141N (knock-in; KI), Hspb8^{+/+} (wild type; WT) mice were housed under the care of the Animal Facility Interfaculty Unit at the University of Antwerp, which is accredited by the Association for Assessment and Accreditation of Laboratory Animals. All Hspb8 mouse experiments were carried out with approval of the Ethical Committee for Laboratory Animals (University of Antwerp). B6;D2-Tg (Eno2-MFN2*R94Q) L51 Ugfm/J (Mitocharch1 or CMT2A Tg) mice were purchased from Jackson Laboratories and are previously described by (Cartoni et al., 2010) and were housed in the animal facility of EPFL. Offspring was genotyped using a standard PCR reaction with the following primers recommended by Jackson Laboratories: 10453: 5'-ATG CAT CCC TTA AGC AC-3' - SEQ ID N° 5, 10454: 5'-CCA GAG GGC AGA ACT TTG-3' - SEQ ID N° 6; oIMR8744: 5'-CAA ATG TTG CTT GTC TGG TG-3' - SEQ ID N° 7, oIMR8745: 5'-GTC AGT CGA GTG CAC AGT TT-3' - SEQ ID N° 8. All experiments on MFN2 mice were done in accordance with Swiss legislation and the European Community Council directive (86/609/EEC) for the care and use of laboratory animals and were approved by the Veterinarian Office of the canton of Vaud and a local ethics committee. All experiments were performed on adult female Hspb8^{K141N/K141N} (knock-in; KI), Hspb8^{+/+} (wild type; WT) mice as described by Bouhy et al. (2017) and on adult male WT or CMT2A Tg mice.

For total protein extraction, cells or sciatic nerves from these mice were lysed with RIPA buffer for 30 min on ice. Protein concentration was quantified with the Bicinchoninic Acid Protein Assay Reagent (Pierce), according to the manufacturer's instructions. Western blotting was performed as described previously (Ydens *et al.,* 2015) using the following antibodies: anti-PFN2 (Sigma-Aldrich), anti-GAMT (Bethyl laboratories), anti-GAPDH (GeneTex) and anti-β-actin (Sigma-Aldrich).

### Statistical analysis

Comparison between the two groups was performed by a Mann-Whitney U-test. Spearman correlation coefficients were used to describe the association between two variables. To evaluate the ability of proteins to predict the disease clinically, receiver operating characteristic (ROC) curves were plotted. Multiple comparisons were performed by one-way analysis of variance (ANOVA) and Bonferroni post hoc multiple comparison test or Dunnett's multiple comparison test. All reported p-values were two-tailed, with a p-value < 0.05 indicating statistical significance. All calculations and statistical analyses were performed with GraphPad Prism version 5.01.

### Results

### Identification of differentially expressed proteins among various CMT2 patients

iTRAQ-based quantitative proteomics were carried out to compare the proteome of lymphoblasts from five selected CMT patients from Belgium (mean age: 51 years, each of the five patients carrying a mutation in a different gene: HSPB8_K141N, RAB7_V162M, NEFL_P8R, HSPB1_R127W, GDAP1_H123R), with pooled samples from six age- and gender-matched unaffected controls (mean age: 56 years). Using Proteome Discoverer 2.0, 16,643 peptides and 4,600 proteins were identified from the mass spectrometry run. The significantly altered proteins from each patient compared to their respective control were analysed (data not shown) and compared to other patients using Vennplex program ⁷.

We found no differentially regulated proteins common to all genotypes. However, two proteins, profilin 2 (PFN2) and guanidinoacetate methyltransferase (GAMT) were found to be downregulated in three out of five genotypes analysed (Figure 1A). These three patients (HSPB8_K141 N, RAB7_V162M and NEFL_P8R) showed an early onset of the symptoms within the first two decades along with a severe phenotype. A clear downregulation of PFN2 and GAMT proteins was also demonstrated by creating a profile plot using the Perseus program (data not shown).

To validate the proteomics data, proteins were isolated from a different passage of the patient's lymphoblast cell lines along with control samples. We also included a lymphoblast cell line from a CMT2A patient (MFN2_R94Q) as CMT2A is the predominant CMT2 subtype characterized by mutations in mitofusin 2 *(MFN2)* ⁸. Western blotting clearly suggested a decrease in both PFN2 and GAMT protein expression in the patient's lymphoblasts compared to the control lines (Figure 1B), thereby confirming the proteomics data.

### PFN2 and GAMT expression is transcriptionally regulated

Next, we studied if the downregulation detected on protein level also corresponded to the change in transcript levels. Remarkably, RT-qPCR analysis of the Belgian cohort demonstrated that the mRNA expression of PFN2 and GAMT was significantly downregulated in the lymphoblast lines from CMT2 patients (n=10) when compared to samples from the healthy individuals described above in the proteomics study (n=6) (p = 0.0047 and p = 0.0002 respectively; Figure 2A, F). This prompted us to expand our CMT cohort to anticipate if these two proteins have relevance as molecular determinants for CMT2. In our extended CMT2 patient cohort (n=43), the expression of PFN2 and GAMT transcripts was significantly downregulated in the patient group compared to the control group comprising 22 unaffected individuals (p = 0.005 and p = 0.01 respectively; Figure 2B, G).

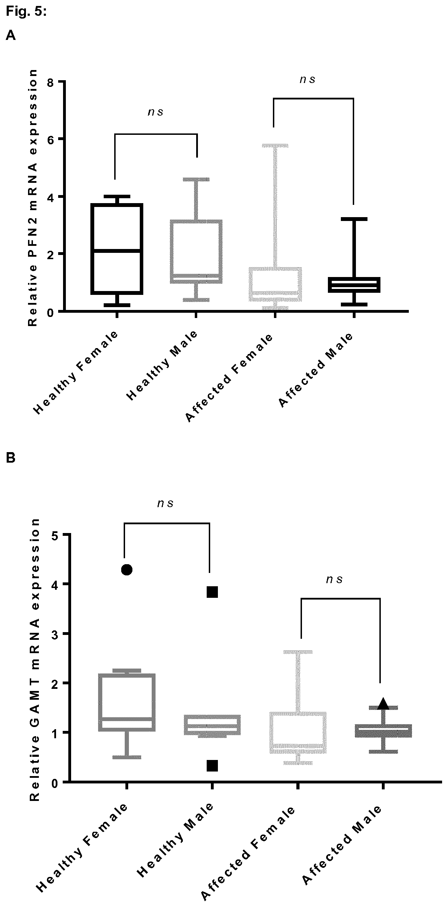

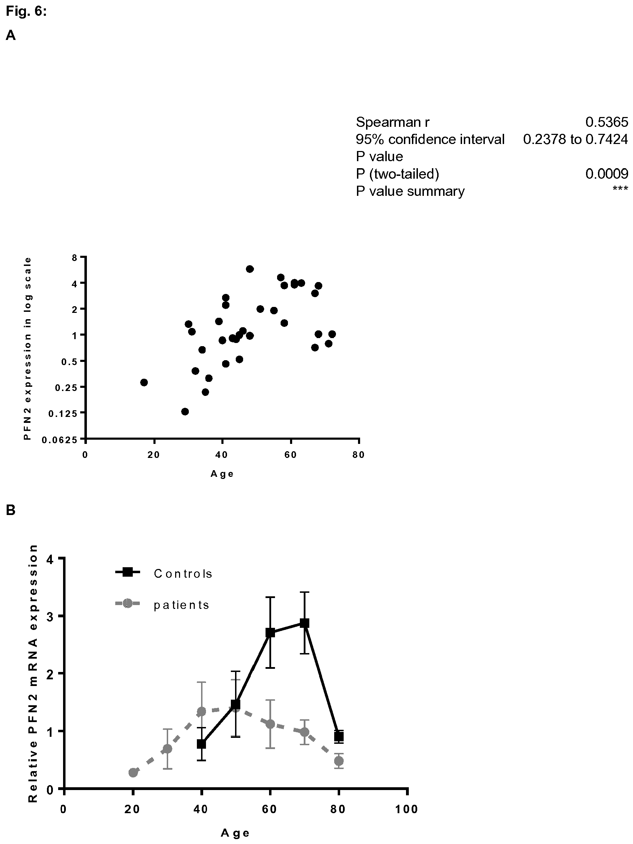

Furthermore, we examined the association of PFN2 and GAMT transcripts with several parameters representing disease and patient sub-groups (age and gender of the patient, affected tissue, symptoms, etc.). No gender-associated differences were observed for PFN2 and GAMT expression (Figure 5). The CMT2 patients with both motor and sensory symptoms (diagnosed as HMSN) showed a severe and significant downregulation in PFN2 (p = 0.003; Figure 2C), whereas PFN2 downregulation did not reach statistical significance in patients presenting only motor deficits (diagnosed as distal hereditary motor neuropathy or dHMN). Additionally, Spearman's rank correlation coefficient (r) suggested a positive correlation between age and PFN2 expression, and an age-dependent progression of PFN2 in the control group (Figure 6A). Strikingly, patients older than 50 years showed a decline in the PFN2 expression while a steep increase was observed in healthy controls older than 50 years (Figure 6B). Thus, we stratified patients based upon age using a mean cut-off of 50 years and found a significant and progressive increase in PFN2 expression in lymphoblasts from healthy control individuals (p = 0.02; Figure 2D), however this increase was not observed in patients. Our data demonstrated a significant decrease in PFN2 expression in patients with age > 50 years as compared to healthy age-matched subjects, which becomes even stronger if we consider only HMSN patients (p = 0.0004 and p < 0.0001, respectively; Figure 2D, E).

For GAMT, not much of a difference was seen upon classifying CMT2 patients based upon HMSN or dHMN (Figure 2H-J). Intriguingly, the significant decrease in PFN2 and GAMT expression could not be correlated with disease severity as measured by CMTES scoring (Figure 7).

### PFN2 and GAMT expression levels as potential biomarkers for CMT2

To assess the clinical significance and diagnostic power of PFN2 and GAMT, we plotted Receiver Operating Characteristic (ROC) curves ⁹. The AUC (Area Under the Curve; a measure of diagnostic power) of PFN2 for the entire cohort was 0.708 and that of GAMT was 0.692 (Figure 3A, E). As expected, the AUC for PFN2 was significantly increased when classifying patients based upon HMSN or dHMN (AUC = 0.7441), age ≥ 50 years (AUC = 0.8318) and particularly for HMSN patients > 50 years of age (AUC = 0.9167), showing a significant association of PFN2 expression with age and the involvement of both motor and sensory deficits (Figure 3B, C, D). No significant difference in AUC for GAMT level was found when classifying patients based upon HMSN or dHMN (AUC = 0.705; data not shown) or age based clustering (AUC = 0.704, Figure 3F), which was comparable to that of the entire patient cohort (0.69). Nevertheless, AUC was highest for GAMT (0.733, Figure 3G) when patients were older than 50 years and had both motor and sensory deficits.

Of note, the Spearman coefficient indicated a positive correlation between GAMT and PFN2 expression, r = 0.78 for healthy individuals and r = 0.55 for all the samples included in this study (Figure 8). Taken together, our results suggest that a decrease in PFN2 expression over time might be an important indicator of CMT2 disease progression, particularly for subjects showing both motor and sensory deficits. In contrast, though GAMT appears to be a marker for axonal CMT, its relationship with disease progression still needs to be explored.

### Relevance of PFN2 and GAMT in the pathogenesis of axonal CMT2

We subsequently investigated the expression of PFN2 and GAMT in differentiated motor neurons obtained from patient-derived iPSC lines. Consistent with the lymphoblast data, we observed a two-fold increase in PFN2 expression (Figure 4A; p = 0.003) in two older control lines (derived from individuals of 50 and 51 years of age) compared to two younger controls (both 24 years old). Upon comparing older patients (HMSN patients, MFN2_R94Q and NEFL_P8R; 50 and 55 years of age) with older controls, we observed a similar two-fold reduction in PFN2 expression as observed in the lymphoblasts (p = 0.002). No significant change was observed for younger patients compared to younger controls (young patient includes dHMN patients with mutations in HSPB8_K141N or HSPB1_G84R; 20 and 40 years old respectively). However, for GAMT, no change in expression was observed (Figure 4B).

Our data suggests a systemic decrease in PFN2 expression in lymphoblasts and motor neurons pinpointing to its possible role in the pathogenesis. However, the major limitation with iPSC technology is that the motor neurons are cultured in an isolated environment unlike the situation in brain and spinal cord and thus may not reflect the real pathology of a specific disorder. Therefore, we also investigated PFN2 expression in the sciatic nerve of a knock-in mouse model for HSPB8 (K141N) mimicking CMT2L/dHMN ¹⁰ and in a transgenic mouse expressing a mutated (R94Q) human mitofusin 2 mimicking CMT2A ¹¹. We found an increase in PFN2 levels over time in the healthy littermate animals (2 months old pre-symptomatic versus 12 months old symptomatic mice) while a decrease in PFN2 expression was detected in post-symptomatic HSPB8 KI mice compared to age-matched healthy littermate controls (n=3 for each group, Figure 4C). In 5 months old CMT2A transgenic mice (n=3 for each group), we observed a tendency towards decrease in PFN2 protein expression in the sciatic nerve compared to healthy littermate controls further supporting the significance of PFN2 in the pathogenesis of the disease (Figure 4C).

### Discussion

Charcot-Marie-Tooth type 2 (CMT2) is an inherited axonal motor and sensory neuropathy exhibiting a vast clinical and genetic heterogeneity thereby complicating its diagnosis and therapeutic intervention. Differential proteomic analysis led to the identification of Profilin 2 (PFN2) and guanidinoacetate methyltransferase (GAMT) as commonly downregulated proteins in the patients compared to age and gender-matched controls. Interestingly, not only the proteins but also their transcripts were found to be significantly altered over controls as determined by RT-qPCR in our multi-center CMT2 cohort.

Profilins are regulators of actin dynamics, which are ubiquitously found in mammals and are essential for the actin polymerization in cells. Two isoforms, PFN1 and PFN2 are present in the brain and play a pivotal role in neurogenesis and synapse formation by interacting with various proteins ^{12, 13}. PFN2 has been recently described as a potential diagnostic biomarker and as a therapeutic target for the treatment of oesophageal squamous cell carcinoma ¹⁴. Intriguingly, altered PFN2 expression has also been described in an intermediate spinal muscular atrophy (SMA) mouse model of axonal degeneration suggesting affected actin dynamics ¹⁵. Albeit, both in SMA and CMT, dysregulated actin-associated proteins eventually alter actin dynamics and thus probably lead to collapse of neuronal networks. The decrease in PFN2 in neurons derived from iPSCs as well as in mouse models suggests its key role in the pathogenesis of CMT besides its role as a prognostic or predictive marker. This is in line with other studies in which the defect in actin-dependent processes is causative of several neurodevelopmental and neurodegenerative diseases thereby further reflecting the importance of a properly regulated cytoskeleton for axonal function ¹⁶. However, it remains possible that besides PFN2, other actin-associated proteins are involved in the disease mechanism leading to an aberrant cytoskeletal dynamics, which warrants further investigation.

GAMT belongs to the creatine biosynthetic pathway that converts guanidinoacetate into creatine. GAMT is crucial for the cellular phosphate associated energy production and storage, particularly in tissues with higher metabolic demands like the brain. GAMT deficiency was found to be implicated in a rare cause of epilepsy ¹⁷. In this study, the difference in expression of GAMT in controls versus patients was rather subtle (though significant) as compared to PFN2. This could be due to the cellular model (lymphoblasts) we have used in this study. We have noticed that unlike for PFN2, lymphocytes show higher expression of GAMT compared to lymphoblasts (data not shown). However, lymphoblasts were used in this study as they offer a continuous source of patient cells with comparable molecular and functional characteristics to their parent lymphocytes thereby eliminating the need of re-sampling ¹⁸. Concomitantly, no differences in GAMT expression was observed in iPSC-derived neurons from CMT2 patients and in mouse sciatic nerves compared to healthy controls. Of note, creatine kinase (CK) levels in serum are routinely measured in clinics for neuromuscular disorders that include muscle weakness and myopathies ¹⁹. Unfortunately, we do not have data on CK levels in our patient cohort. It remains likely that the elevated levels of CK in these patients give rise to reduced GAMT levels and is indicative of muscle weakness in CMT patients. However, it remains interesting to investigate the expression of GAMT in patient's serum, and using other CMT2 mouse models, in the muscle and liver where GAMT is primarily expressed.

To investigate the feasibility of using PFN2 and GAMT as a clinical diagnostic biomarker of CMT2, ROC curves were plotted to determine the specificity, sensitivity and AUC score. We acquired similar or even higher sensitivity, specificity and AUC values compared to traditional AD biomarkers Aβ42 and tau (AUC= 0.755 and 0.78 respectively)²⁰, upon classifying patients based on their age (>50y) and symptoms (both motor and sensory symptoms involved) underscoring the importance of PFN2 as a novel diagnostic biomarker. In addition, we found that PFN2 expression gradually increases with age in healthy subjects, in contrast to, CMT2 patients, in which PFN2 expression progressively decreases with age. This suggests that PFN2 expression may serve as a potential biomarker of the severity and progression of CMT2 neuropathy. It was intriguing that 3 out of 5 lymphoblast samples showed the decrease in PFN2 and GAMT in the initial proteomic analysis. Data generated on the expanded cohort revealed that HSPB1 samples did not replicate this decrease probably because mutations in HSPB1 mostly give rise to pure hereditary motor neuropathies, whereas for GDAP1 mutations, the results were sample specific as other GDAP patients recruited in the extended cohort clearly exhibited the decrease in PFN2 and GAMT. It must be pointed out that we detected PFN2 expression as a whole and did not further distinguish its two isoforms. It has been reported that the PFN2a isoform is present in the majority of cells including neurons, whereas PFN2b is a minor splice product ²¹. For GAMT, further classification did not significantly improve the sensitivity or specificity, though AUC values were highest when classifying patients based on age and involvement of both sensory and motor symptoms.

Taken together, we initiated this study based on our hypothesis that pathophysiological pathways encompassing multiple forms of CMT2 are likely to implicate similar underlying mechanisms. This led to the identification of candidates PFN2 and GAMT implicated as "signature" molecules in multiple CMT2 subtypes. To our knowledge, this is the first study involving multiple CMT2 causative genes at once and reporting biomarkers for CMT2. Our results highlight that measuring PFN2 and GAMT may help clinicians to monitor disease activity and progression, and may guide potential therapeutic strategies.

### REFERENCES

1. Saporta MA, Shy ME. Inherited peripheral neuropathies. Neurologic Clinics 2013;31:597-619.
2. Rossor AM, Polke JM, Houlden H, Reilly MM. Clinical implications of genetic advances in Charcot-Marie-Tooth disease. Nature Reviews Neurology 2013;9:562-571.
3. Pareyson D, Marchesi C. Diagnosis, natural history, and management of Charcot-Marie-Tooth disease. The Lancet Neurology 2009;8:654-667.
4. Soldevilla B, Cuevas-Martin C, Ibanez C, et al. Plasma metabolome and skin proteins in Charcot-Marie-Tooth 1A patients. PloS one 2017;12:e0178376.
5. Fledrich R, Mannil M, Leha A, et al. Biomarkers predict outcome in Charcot- Marie-Tooth disease 1A. Journal of Neurology, Neurosurgery, and Psychiatry 2017.
6. Bouhy D, Timmerman V. Animal models and therapeutic prospects for Charcot-Marie-Tooth disease. Annals of Neurology 2013;74:391-396.
7. Cai H, Chen H, Yi T, et al. VennPlex--a novel Venn diagram program for comparing and visualizing datasets with differentially regulated datapoints. PloS one 2013;8:e53388.
8. Timmerman V, Strickland AV, Zuchner S. Genetics of Charcot-Marie-Tooth (CMT) Disease within the Frame of the Human Genome Project Success. Genes 2014;5:13-32.
9. Hajian-Tilaki K. Receiver Operating Characteristic (ROC) Curve Analysis for Medical Diagnostic Test Evaluation. Caspian Journal of Internal Medicine 2013;4:627-635.
10.Bouhy D, Juneja M, Katona I, et al. A knock-in/knock-out mouse model of HSPB8-associated distal hereditary motor neuropathy and myopathy reveals toxic gain-of-function of mutant Hspb8. Acta Neuropathologica 2017.
11.Cartoni R, Arnaud E, Medard JJ, et al. Expression of mitofusin 2(R94Q) in a transgenic mouse leads to Charcot-Marie-Tooth neuropathy type 2A. Brain : a journal of neurology 2010;133:1460-1469.
12.Wang YY, Wu HI, Hsu WL, et al. In vitro growth conditions and development affect differential distributions of RNA in axonal growth cones and shafts of cultured rat hippocampal neurons. Molecular and Cellular Neurosciences 2014;61:141-151.
13.Murk K, Wittenmayer N, Michaelsen-Preusse K, et al. Neuronal profilin isoforms are addressed by different signalling pathways. PloS one 2012;7:e34167.
14.Cui XB, Zhang SM, Xu YX, et al. PFN2, a novel marker of unfavorable prognosis, is a potential therapeutic target involved in esophageal squamous cell carcinoma. Journal of Translational Medicine 2016;14:137.
15.Bowerman M, Anderson CL, Beauvais A, Boyl PP, Witke W, Kothary R. SMN, profilin Ila and plastin 3: a link between the deregulation of actin dynamics and SMA pathogenesis. Molecular and Cellular Neurosciences 2009;42:66-74.
16.Kevenaar JT, Hoogenraad CC. The axonal cytoskeleton: from organization to function. Frontiers in Molecular Neuroscience 2015;8:44.
17.Stern WM, Winston JS, Murphy E, Cross JH, Sander JW. Guanidinoacetate methyltransferase (GAMT) deficiency: a rare but treatable epilepsy. Practical Neurology 2017;17:207-211.
18.Hussain T, Mulherkar R. Lymphoblastoid Cell lines: a Continuous in Vitro Source of Cells to Study Carcinogen Sensitivity and DNA Repair. International Journal of Molecular and Cellular Medicine 2012;1:75-87.
19.Moghadam-Kia S, Oddis CV, Aggarwal R. Approach to asymptomatic creatine kinase elevation. Cleveland Clinic Journal of Medicine 2016;83:37-42.
20.Craig-Schapiro R, Kuhn M, Xiong C, et al. Multiplexed immunoassay panel identifies novel CSF biomarkers for Alzheimer's disease diagnosis and prognosis. PloS one 2011 ;6:e18850.
21.Di Nardo A, Gareus R, Kwiatkowski D, Witke W. Alternative splicing of the mouse profilin II gene generates functionally different profilin isoforms. Journal of Cell Science 2000;113 Pt 21:3795-3803.

## Claims

1. A method for the diagnosis of Charcot-Marie-Tooth type 2 disease in a subject; said method comprising the steps of:
1) determining the expression level of one or more biomarkers selected from the list comprising: Profilin 2 (PFN2) and guanidinoacetate methyltransferase (GAMT), in a test sample obtained from said subject;
2) comparing said expression level of said one or more biomarkers as determined in step 1) with the expression level of said one or more biomarkers in a reference sample;
3) wherein a reduced expression level of said one or more biomarkers in said test sample compared to said reference sample is indicative of said subject suffering from Charcot-Marie-Tooth type 2 disease.

2. The method according to claim 1; wherein said samples are selected from the list comprising a lymphoblast sample or a motor neuron sample obtained from subject-derived iPSC lines.

3. The method according to anyone of claims 1-2; wherein said samples are obtained from an individual having an age of at least 50 years.

4. The method according to anyone of claims 1-3; wherein said Charcot-Marie-Tooth type 2 disease is selected from the following subtypes: patients diagnosed with hereditary motor and sensory neuropathy (HMSN); or patients with predominantly motor involvement, known as distal hereditary motor neuropathy (dHMN).

5. The method according to anyone of claims 1-4; wherein said test sample is a motor neuron sample obtained from a subject-derived iPSC line, and said biomarker is selected to be PFN2.

6. The method according to anyone of claims 1-4; wherein said test sample is a lymphoblast sample, and said biomarker is selected to be PFN2.

7. The method according to anyone of claims 1-4; wherein said test sample is a lymphoblast sample, and said biomarker is selected to be GAMT.

8. The method according to anyone of claims 1-4; wherein said test sample is a serum sample, and said biomarker is selected to be PFN2 or GAMT.

9. In-vitro use in a method according to claim 1 of a kit for diagnosing Charcot-Marie-Tooth type 2 disease in a subject the kit comprising means for determining the expression level of one or more biomarkers selected from the list comprising: Profilin 2 (PFN2) and guanidinoacetate methyltransferase (GAMT).

10. The method according to anyone of claims 1-8 or the use according to claim 9; wherein said expression level is determined from protein or mRNA sample.

11. A kit comprising primers for determining the expression level of PFN2 and GAMT by qRT-PCR.

## Patentansprüche

1. Ein Verfahren zur Diagnose der Charcot-Marie-Tooth-Erkrankung vom Typ 2 an einer Versuchsperson; wobei das Verfahren die folgenden Schritte aufweist:
1) Bestimmen des Expressionsniveaus eines oder mehrerer Biomarker ausgewählt aus der Liste, die folgendes aufweist: Profilin 2 (PFN2) und Guanidinoacetat-Methyltransferase (GAMT), in einer der Versuchsperson entnommenen Testprobe;
2) Vergleichen des Expressionsniveaus des einen oder der mehreren in Schritt 1) bestimmten Biomarker mit dem Expressionsniveau des einen oder der mehreren Biomarker in einer Referenzprobe;
3) wobei ein verringertes Expressionsniveau des einen oder der mehreren Biomarker in der Testprobe im Vergleich zu der Referenzprobe darauf hinweist, dass die Versuchsperson an der Charcot-Marie-Tooth-Erkrankung vom Typ 2 leidet.

2. Das Verfahren nach Anspruch 1; wobei die Proben aus der Liste ausgewählt sind, die eine Lymphoblastenprobe oder eine Motoneuronenprobe aufweist, welche aus von Versuchspersonen stammenden iPSC-Linien entnommen werden.

3. Das Verfahren nach einem der Ansprüche 1 bis 2; wobei die Proben einer Person mit einem Alter von mindestens 50 Jahren entnommen werden.

4. Das Verfahren nach einem der Ansprüche 1 bis 3; wobei die Charcot-Marie-Tooth-Erkrankung vom Typ 2 aus den folgenden Subtypen ausgewählt ist: Patienten, bei denen hereditär motorisch-sensorische Neuropathie (HMSN) diagnostiziert ist; oder Patienten mit vorwiegend motorischer Beteiligung, bekannt als distale hereditäre motorische Neuropathie (dHMN).

5. Das Verfahren nach einem der Ansprüche 1 bis 4; wobei die Testprobe eine Motoneuronenprobe ist, die einer von einer Versuchsperson stammenden iPSC-Linie entnommen wird, und der Biomarker als PFN2 ausgewählt wird.

6. Das Verfahren nach einem der Ansprüche 1 bis 4; wobei die Testprobe eine Lymphoblastenprobe ist und der Biomarker als PFN2 ausgewählt wird.

7. Das Verfahren nach einem der Ansprüche 1 bis 4; wobei die Testprobe eine Lymphoblastenprobe ist und der Biomarker als GAMT ausgewählt wird.

8. Das Verfahren nach einem der Ansprüche 1 bis 4; wobei die Testprobe eine Serumprobe ist und der Biomarker als PFN2 oder GAMT ausgewählt wird.

9. In-vitro Verwendung, in einem Verfahren nach Anspruch 1, eines Kits zur Diagnose der Charcot-Marie-Tooth-Erkrankung vom Typ 2 bei einer Versuchsperson, wobei das Kit Mittel zur Bestimmung des Expressionsniveaus eines oder mehrerer Biomarker aufweist, die aus der Liste mit folgendem ausgewählt sind: Profilin 2 (PFN2) und Guanidinoacetat-Methyltransferase (GAMT).

10. Das Verfahren nach einem der Ansprüche 1 bis 8 oder die Verwendung nach Anspruch 9; wobei das Expressionsniveau aus einer Protein- oder mRNA-Probe bestimmt wird.

11. Ein Kit mit Primern zur Bestimmung des Expressionsniveaus von PFN2 und GAMT durch qRT-PCR.

## Revendications

1. Procédé de diagnostic de la maladie de Charcot-Marie Tooth du type 2 chez un sujet ; ledit procédé comprenant les étapes de :
1) détermination du niveau d'expression d'un ou plusieurs biomarqueurs sélectionnés parmi la liste comprenant :
la profiline 2 (PFN2) et la guanidinoacétate méthyltransférase (GAMT), dans un échantillon de test obtenu à partir dudit sujet ;
2) comparaison dudit niveau d'expression desdits un ou plusieurs biomarqueurs tel que déterminé dans l'étape 1) au niveau d'expression desdits un ou plusieurs biomarqueurs dans un échantillon de référence ;
3) dans lequel un niveau d'expression réduit desdits un ou plusieurs biomarqueurs dans ledit échantillon de test comparé audit échantillon de référence est représentatif du fait que ledit sujet souffre de la maladie de Charcot-Marie Tooth du type 2.

2. Procédé selon la revendication 1 ; dans lequel lesdits échantillons sont sélectionnés à partir de la liste comprenant un échantillon de lymphoblaste ou un échantillon de neurone moteur obtenu à partir de lignées iPSC dérivées du sujet.

3. Procédé selon l'une quelconque des revendications 1 à 2 ; dans lequel lesdits échantillons sont obtenus à partir d'un individu présentant un âge d'au moins 50 ans.

4. Procédé selon l'une quelconque des revendications 1 à 3 ; dans lequel ladite maladie de Charcot-Marie Tooth du type 2 est sélectionnée à partir des sous-types suivants : des patients diagnostiqués avec une neuropathie motrice et sensorielle héréditaire (HMSN) ; ou des patients présentant une atteinte principalement motrice, connue en tant que neuropathie motrice héréditaire distale (dHMN).

5. Procédé selon l'une quelconque des revendications 1 à 4 ; dans lequel ledit échantillon de test est un échantillon de moto neurone obtenu à partir d'une lignée iPSC dérivées du sujet et le biomarqueur est sélectionné de manière à être la profiline PFN2.

6. Procédé selon l'une quelconque des revendications 1 à 4 ; dans lequel ledit échantillon de test est un échantillon de lymphoblaste et ledit biomarqueur est sélectionné de manière à être la profiline PFN2.

7. Procédé selon l'une quelconque des revendications 1 à 4 ; dans lequel ledit échantillon de test est un échantillon de lymphoblaste et ledit biomarqueur est sélectionné de manière à être l'enzyme GAMT.

8. Procédé selon l'une quelconque des revendications 1 à 4 ; dans lequel ledit échantillon de test est un échantillon de sérum et ledit biomarqueur est sélectionné de manière à être le gène PFN2 ou l'enzyme GAMT.

9. Utilisation in vitro dans un procédé selon la revendication 1 d'un kit destiné à diagnostiquer la maladie de Charcot-Marie Tooth du type 2 chez un sujet, le kit comprenant des moyens de détermination du niveau d'expression d'un ou plusieurs biomarqueurs sélectionnés à partir de la liste comprenant : la profiline 2 (PFN2) et la guanidinoacétate méthyltransférase (GAMT).

10. Procédé selon l'une quelconque des revendications 1 à 8 ou utilisation selon la revendication 9 ; dans lequel ledit niveau d'expression est déterminé à partir d'une protéine ou d'un échantillon d'ARNm.

11. Kit comprenant des amorces afin de déterminer le niveau d'expression de PFN2 et de GAMT par qRT-PCR.
